# FASCICULE DE BREVET EUROPEEN

(11) **EP 0 502 765 B1**
(45) Date de publication et mention de la délivrance du brevet: **06.11.1996**
(21) Numéro de dépôt: 92400519.2
(22) Date de dépôt: 28.02.1992
(51) Int. Cl.: A61K 31/685, A61K 31/66, A61K 33/06

(54) **Nouvelles compositions diététiques à base de lipides complexes phosphorés et leur utilisation dans les troubles du sommeil**
Neue, dietätische Zubereitungen auf Basis von Phosphor-Lipidkomplexen sowie deren Verwendung bei Schlafstörungen
New dietary compositions based on phosphorylated lipid complexes and their use in sleeping disorders

(30) Priorité: 05.03.1991 FR 9102612
(43) Date de publication de la demande: 09.09.1992
(73) Titulaire: INSTITUT DE RECHERCHE BIOLOGIQUE S.A., F-78870 Bailly (FR)
(72) Inventeur: Ponroy, Yves, F-78890 Garancieres (FR); Forgeot, Marcel, F-78720 Dampierre en Yvelines (FR); Fraisse, Pierrette, F-34000 Montpeller (FR)
(74) Mandataire: Burtin, Jean-François

(56) Documents cités:
- EP-A- 0 148 045
- EP-A- 0 217 258
- EP-A- 0 347 843
- EP-A- 0 396 081
- DE-A- 3 517 916
- FR-A- 2 301 244
- FR-A- 2 649 322

## Description

La présente invention a pour objet une nouvelle utilisation de compositions destinées à la thérapeutique diététique, à base de lipides complexes phosphorés.

Elle a plus particulièrement pour objet l'utilisation de nouvelles compositions destinées à la thérapeutique diététique, en vue de la réalisation d'une préparation combattant les effets du stress et de la fatigue nerveuse et de la suppression du besoin en anxiolytiques, en tranquillisants et/ou en hypnotiques par administration de lipides complexes phosphorés chez de tels sujets.

On sait, en effet, que les symptômes de la fatigue nerveuse se traduisent par des phénomènes d'asthénie, de tristesse, de manque d'entrain, par un manque de clarté dans l'idéation ou par des troubles de la mémoire qui s'avère facilement défaillante.

La fatigue nerveuse résulte en grande partie de la difficulté que connaissent beaucoup de sujets à s'adapter aux tensions et aux contraintes de la vie professionnelle ou familiale. Elle est dûe à la quantité très importante d'énergie qu'il faut dépenser pour se protéger contre les soucis de la vie et beaucoup de gens sont incapables de résister à cette pression permanente. La fatigue nerveuse les laisse épuisés avec un manque total de dynamisme ou de réaction.

Une phase ultérieure, proche de l'état pathologique, se situe dans les troubles du sommeil. Le sujet agité ou perturbé connait une difficulté à l'endormissement ou une tendance au réveil au milieu de la nuit sans pouvoir se rendormir avant le matin.

La tentation alors est grande d'avoir recours à des somnifères ou à des inducteurs de sommeil pour remédier à ces troubles. C'est ce qui explique le succès des hypnotiques tels que les barbituriques ou les quinazolones (Methaqualone ou Mecloqualone). C'est ce qui explique aussi le recours très fréquents de sujets soumis à des stress, aux médicaments du type Benzodiazépine ou pyrrolopyridines qui facilitent l'endormissement ou qui assurent des nuits calmes et détendues. Malheureusement, le défaut de beaucoup de ces produits est de développer des phénomènes d'accoutumance entrainant une situation de dépendance ou de besoin de doses plus élevées par souci d'obtenir un résultat certain et rapide. On constate donc que des sujets à psychisme fragile comme des personnes âgées consomment des hypnotiques ou des tranquillisants sur des périodes de temps considérables allant par exemple de 1 mois à 10 ans.

On constate aussi dans certains cas que ces sujets associent un hypnotique et un ou plusieurs tranquillisants pour être bien certains de trouver le sommeil et passer une nuit complète en état hypnotique.

Cette accoutumance et ce recours systématique à des substances psychotropes est un phénomène social grave par suite des détériorations psychiques sérieuses qui en résultent et qui sont susceptibles de modifier profondément le comportement.

Le corps médical a pris conscience de ce danger et s'efforce de procéder à l'arrêt de telles médications. Ces tentatives sont rarement couronnées de succès en raison de ce que l'on appelle le "syndrome de sevrage" au cours duquel le sujet sevré d'hypnotique ou de tranquillisant traverse une crise grave de dépression, de confusion psychique ou d'agitation comparable aux états de privation des morphiniques chez des sujets habitués à ingérer des drogues contenant des dérivés de la morphine.

On a trouvé maintenant et c'est ce qui constitue l'objet de la présente invention qu'une préparation de thérapeutique diététique renfermant essentiellement des phospholipides cérébraux à très longue chaine d'acides gras poly insaturés, associés ou non à de la Vitamine E et/ou à des sels de magnésium pouvait constituer un moyen efficace de lutter contre les phénomènes dépressifs liés au sevrage ou aux pertes de sommeil en l'absence d'hypnotique.

Le rôle des phospholipides cérébraux dans la protection des membranes des neurones est déjà connu. Il s'agit dans la présente invention d'un nouvel emploi de ces phospholipides qui permet de supprimer complètement ou principalement l'administration de somnifère ou de tranquillisant d'une manière progressive, durable et efficace.

Spécifiquement, l'invention a pour objet l'utilisation de compositions diététiques destinées à la thérapeutique diététique caractérisée en ce que la composition contient comme constituant principal des triglycérides phosphorylés de formule : 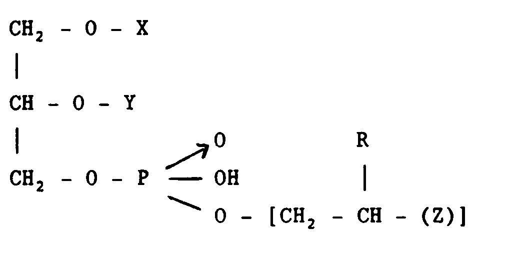 dans laquelle X et Y, identiques ou différents, représentent le reste acyle d'un acide gras polyinsaturé, ayant de 18 à 24 atomes de carbone Z représente un radical aminé primaire, secondaire, tertiaire ou quaternaire
et R représente de l'hydrogène ou un carboxyle

Parmi les triglycérides phosphorylés on pourra citer ceux pour lesquels X et/ou Y sont des acides tri, penta ou hexa ethyléniques comme un reste d'acide docosahexenoïque.

Parmi les restes hydroxylés fixés sur l'acide phosphorique, on pourra citer l'éthanolamine (Z : NH₂ R = H) la sérine (Z = NH₂ R = COOH)
- l'inositol
- la carnitine
- la choline
   Z = N (CH₃)₄
   R = H
   et n = 1

Les restes d'acide gras sont fréquemment identiques mais il existe aussi la possibilité d'utiliser des triglycérides mixtes où les acides gras sont différents.

Ces glycérides phosphorylés sont en règle générale d'origine animale et leur principale source est un produit d'extraction des tissus cérébraux humains ou animaux. Il n'est cependant pas exclu que des glycérides phosphorylés de composition similaire se trouvent dans les végétaux.

La teneur de ces glycérides phosphorylés dans les compositions diététiques de l'invention peut être définie par la teneur en lipides qui varie de 10 à 30 g pour 100 g de composition et de préférence de 18 à 22 g. soit de 0,005 g à 0,100 g de glycérides phosphorylés, par prise unitaire.

On a constaté également que la présence simultanée de Tocophérols jouait un rôle favorable en renforçant l'efficacité des glycérides phosphorylés, en s'opposant ou en limitant leur oxydation dans l'organisme ou en captant les radicaux libres qui favorisent l'hydroxy-peroxydation des acides gras polyinsaturés à longue chaine. Les tocophérols peuvent être additionnés sous forme d'huile végétale concentrée titrant 25% d'α-tocophérol et/ou sous forme d'α-tocophérol acétate protégé par un enrobage à 50%.
D'une manière préférée, la teneur en tocophérols s'échelonne quelqu'en soit la forme de 0,7 à 1,4 g pour 100 g de préparation.

En outre, l'adjonction de magnésium sous forme de sels solubles ou d'oxyde peu soluble, à la composition diététique selon l'invention, joue un rôle favorable en stimulant le métabolisme de l'organisme et en provoquant une action psycho-sédative et équilibrante. La concentration en oxyde de magnésium dans les compositions selon l'invention s'échelonne entre 25 et 35 g pour 100 g de préparation.

Les préparations de thérapeutique diététique selon l'invention contiennent en outre des agents diluants, des excipients inertes et des protéines alimentaires.

La posologie journalière est variable selon l'ancienneté de la consommation des hypnotiques ou des tranquillisants et de l'efficacité de l'administration post-sevrage.

Elle est en général de 2 à 6 prises par jour de préférence de 3 prises unitaires ingérées au moment du repas du soir.

La durée de l'administration est d'une manière préférée de plusieurs mois, et plus précisement de 1 à 4 mois. L'efficacité des compositions diététiques selon l'invention va en augmentant avec la durée de l'administration. Elle est déjà bonne après 30 jours d'administration. Elle est optimale après 60 jours d'administration. Elle se maintient égale par la suite.

### EXEMPLE D'UNE PREPARATION DE THERAPEUTIQUE DIETETIQUE SELON L'INVENTION :

- phospholipides d'origine cérébrale
- concentré d'huile de germe de blé
- bromure de magnésium
- lactose
- lactalbumine
- caseinates
- phosphate tricalcique
- maltodextrine
pour une gélule de 400 mg environ.

### ETUDE DES PREPARATIONS DE THERAPEUTIQUE DIETETIQUE SELON L'INVENTION SUR LE TEMPS DE SOMMEIL.

Une préparation de thérapeutique diététique selon l'invention a été expérimentée sur des volontaires des deux sexes âgés de 18 à 60 et prenant régulièrement des hypnogènes ou des neuroleptiques depuis plus de trois mois en dehors de toute prescription médicale.

Les volontaires ont été invités à remplir un questionnaire aux Jours J₀, J₁₅, J₃₀, J₄₅ et J₆₀. Ils devaient indiquer la durée du sommeil du sujet le jour correspondant ainsi que le délai d'endormissement et la consommation éventuelle de neuroleptiques ou d'hypnogènes.

La population traitée était de 60 personnes d'âge moyen 42 ans (41 femmes, 19 hommes) ayant une insomnie durant en moyenne depuis 36 ± 54 mois. La posologie était de 3 à 4 gélules par jour avec en moyenne 3 par jour. La durée du traitement a été de deux mois.

### RESULTATS

L'essai entrepris sur ces volontaires a permis d'établir que dès la 2ème semaine 52 % des sujets se passaient complètement de leur anxiolytique ou de leur hypnogène, que cette proportion allait en croissant avec le temps et qu'après 2 mois de traitement il n'y avait plus que 23% des sujets qui continuaient à utiliser des drogues.

Dans le même temps, le délai moyen d'endormissement a diminué de 84 à 38 mn et la durée du temps de sommeil a augmenté de 1 h 50. Ces résultats sont très significatifs sur le plan statistique.

Dans une autre expérience, le sevrage partiel a été obtenu chez 28 sujets (25%) et le sevrage total a été obtenu dans un délai de 42 jours en moyenne chez 55% des sujets.

Ces résultats sont très encourageants compte tenu du fait que chez certains sujets prenant préalablement des benzodiazépines à ½ vie courte le sevrage s'est avéré impossible.

Le sevrage également s'est avéré plus difficile chez les hommes d'une part et chez les sujets présentant une anxiété secondaire d'autre part.

La consommation d'alcool et/ou de tabac est en moyenne plus élevée chez les sujets sevrés que chez les sujets non sevrés.

Les échecs quant il y en a eu ont été plus nombreux chez des sujets présentant une insomnie installée très récemment et chez les sujets utilisant des benzodiazépines à ½ vie courte.

La tolérance du traitement a été excellente chez tous les sujets. La qualité du sommeil a été jugée très satisfaisante.

Dans un troisième essai sur 40 sujets volontaires, âgés de 60 ans et plus, avec une moyenne de 67,33 ± 7,8 ans en majorité des femmes, on a obtenu les résultats suivants :

### ETUDE DU SOMMEIL

### a) Durée d'endormissement

Son évolution au cours de l'administration d'une composition selon l'invention a été la suivante :

| | JO | J15 | J30 | J45 | J60 |
|---|---|---|---|---|---|
| n | 40 | 40 | 40 | 40 | 40 |
| m (mn) | 71,50 | 64,00 | 60,25 | 58,50 | 58,50 |
| +/- SD | 10,44 | 8,89 | 8,21 | 9,10 | 9,10 |

On constate une diminution du temps d'endormissement des sujets très marquée au début de l'essai, progressive jusqu'au 45ème jour, puis stable entre le 45ème et le 60ème jour.
Cette diminution est statistiquement hautement significative par rapport aux valeurs de base à l'entrée dans l'essai et ce, dès la première quinzaine de l'administration de la composition (t = 6,98 ; ddl = 39 ; p < 0,001).

### b) Durée du sommeil

Son évolution au cours de l'administration d'une composition selon l'invention a été la suivante :

| | JO | J15 | J30 | J45 | J60 |
|---|---|---|---|---|---|
| n | 40 | 40 | 40 | 40 | 40 |
| m (heures) | 5,11 | 6,02 | 6,52 | 6,69 | 6,76 |
| +/- SD | 0,54 | 0,87 | 1,03 | 1,12 | 1,15 |

On constate une augmentation de la durée du sommeil des sujets très marquée au début de l'essai puis progressive jusqu'à la fin du traitement : gain total 1 h 39 en moyenne.
Cette augmentation est statistiquement hautement significative par rapport aux valeurs de base à l'entrée dans l'essai et ce, dès la première quinzaine d'administration de la composition (t = 9,03 ; ddl = 39 ; P < 0,001).

Dans les deux tableaux qui précèdent, l'abrévation n indique le nombre de sujets, m et mn le temps d'endormissement mesuré, SD la déviation standard sur les résultats, ddl le degré de liberté, t le facteur du test de student, p le degré de probabilité.

Ces résultats sont à mettre en parallèle avec la suppression des hypnotiques chez les patients.

Ainsi, la durée du sommeil augmente pendant toute la durée de l'essai malgré un sevrage de 65% des sujets en fin d'étude.

Les compositions selon l'invention ont donc permis, chez nos sujets, d'améliorer nettement la durée du sommeil alors que dans le même temps, les hypnotiques étaient supprimés pour un grand nombre de patients.

Il est à remarquer que parmi les 14 sujets n'ayant pas supprimé leur traitement par les benzodiazépines ou ne l'ayant supprimé que partiellement, 5 ont constaté une amélioration de leur sommeil portant sur le délai d'endormissement et sur la durée.

## Revendications

1. Utilisation d'une composition renfermant des triglycérides phosphorylés de formule 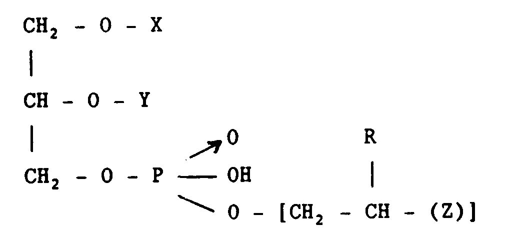 dans laquelle X et Y, identiques ou différents, représentent le reste acyle d'un acide gras polyinsaturé ayant de 18 à 24 atomes de carbone
Z représente un radical aminé,primaire, secondaire, tertiaire ou quaternaire
et R représente de l'hydrogène ou un carboxyle
en association ou en mélange avec des diluants, des excipients, des agents liants inertes et des compléments alimentaires
en vue de la réalisation d'une préparation de thérapeutique diététique destinée à supprimer le besoin en anxiolitiques, en tranquillisants et/ou en hypnotiques, chez des sujets manifestant de la fatigue nerveuse.

2. Utilisation d'une préparation de thérapeutique diététique selon la revendication 1° dans laquelle X et/ou Y sont les restes acyle d'un acide gras insaturé ayant 6 doubles liaisons.

3. Utilisation d'une préparation de thérapeutique diététique selon la revendication 1° dans laquelle le triglycéride phosphorylé est un triglycéride phosphorylé obtenu à partir de cervelles animales.

4. Utilisation d'une préparation de thérapeutique diététique selon l'une des revendications 1 à 3° dans laquelle la teneur en glycéride phosphorylé s'échelonne de 0,005 g à 0,100 g par prise unitaire.

5. Utilisation d'une préparation de thérapeutique diététique selon l'une des revendications 1 à 4° dans laquelle les matières inertes sont celles qui conviennent pour l'administration par voie digestive.

6. Utilisation d'une préparation de thérapeutique diététique selon l'une des revendications 1 à 5° caractérisée en ce que la préparation de thérapeutique diététique renferme en outre des tocophérols.

7. Utilisation d'une préparation de thérapeutique diététique selon l'une des revendications 1 à 6° dans laquelle la teneur de la composition en tocophérols s'échelonne de 0,7 g à 1,4 g.

8. Utilisation d'une préparation de thérapeutique diététique selon l'une des revendications 1 à 7° caractérisée en ce que la préparation renferme en outre des sels de magnésium.

9. Utilisation d'une préparation de thérapeutique diététique selon l'une des revendications 1 à 7° dans laquelle la teneur en sel de magnésium exprimée en oxyde s'échelonne de 25 à 35 g jour 100 g.

10. Utilisation d'une préparation de thérapeutique diététique selon l'une des revendications précédentes dans laquelle l'administration journalière s'échelonne de 2 à 6 prises unitaires chez l'homme adulte.

## Claims

1. Use of a composition containing phosphorylated triglycerides having the formula 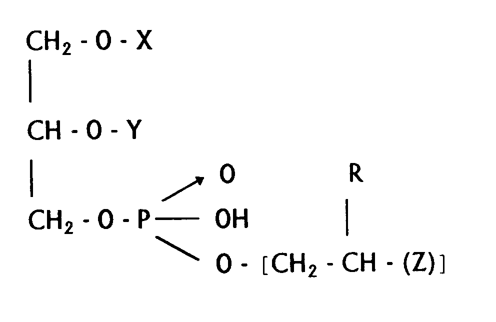 in which X and Y, the same or different, represent the acyl moiety of a polyunsaturated fatty acid having from 18 to 24 carbon atoms
Z is a primary, secondary, tertiary or quaternary amino radical
and R is a hydrogen or a carboxyl
in conjunction or in admixture with diluents, carriers, inert binding agents and nutritionals complements
for the realization of a preparation for dietetic therapy intended to curb the need in anxiolytics, tranquillizers and/or hypnotics, in patients demonstrating nervous tiredness.

2. Use of a preparation for dietetic therapy according to claim 1, wherein X and/or Y are acyl moieties of an unsaturated fatty acid having 6 double bonds.

3. Use of a preparation for dietetic therapy according to claim 1, wherein the phosphorylated triglyceride is a phosphorylated triglyceride obtained from animal's brains.

4. Use of a preparation for dietetic therapy according to any of claims 1 to 3, wherein the content in phosphorylated glyceride ranges from 0,005 g to 0,100 g per unit dosage.

5. Use of a preparation for dietetic therapy according to any of claims 1 to 4, wherein the inert matters are those which are suitable for the digestive way.

6. Use of a preparation for dietetic therapy according to any of claims 1 to 5, wherein the dietetic therapeutic preparation moreover contains tocopherols.

7. Use of a preparation for dietetic therapy according to any of claims 1 to 6, wherein the content of the composition in tocopherols ranges from 0,7 g to 1,4 g.

8. Use of a preparation for dietetic therapy according to any of claims 1 to 7, wherein the preparation moreover contains magnesium salts.

9. Use of a preparation for dietetic therapy according to any of claims 1 to 7, wherein the content in magnesium salt expressed in oxide, ranges from 25 to 35 g for 100 g.

10. Use of a preparation for dietetic therapy according to any of previous claims, wherein the daily administration ranges from 2 to 6 unit dosages in the adult man.

## Patentansprüche

1. Verwendung von einer Zusammensetzung aus phosphorhaltende Triglyzerid, der Formel 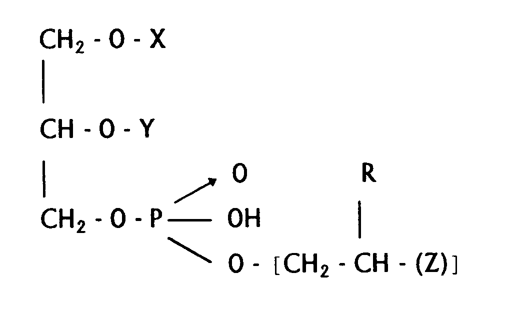 in der X und Y, identische oder unterschiedliche sind, für eine Acylrest von einer polyungesättigte Fettsäure mit 18 bis 24 Kohlenstoff Atomen steht
Z einen primären, sekundären, tertiären oder quaternären Aminoradikal ist
R einen Wasserstoffatom oder eine Carbonsäure ist
in Verbindung oder Vermischung mit einen Verdünnungsmittel, einen Träger, einen inerten Bindungsmittel, und einen Ernährungsergänzungsmittel
für die Herstellung einer diätetischen Zubereitung für therapeutische Zweck die das Bedürfnis für Ängsthemmendemittel, Beruhigungsmittel und/oder Hypnotische bei Personen die nervöse Müdigkeit aufweisen, zu unterdrücken bestimmt sind.

2. Verwendung von einer diätetische Zubereitung für therapeutische Zweck nach Anspruch 1, worin X und/oder Y für Acylrest von einer ungesättigte Fettsaüre mit 6 Doppelbindungen steht.

3. Verwendung von einer diätetische Zubereitung für therapeutische Zweck nach Anspruch 1, worin das phosphorhaltende Triglyzerid, ein phosphorhaltende Triglyzerid von tierischen Gehirne stammt.

4. Verwendung von einer diätetische Zubereitung für therapeutische Zweck nach einer der Ansprüche 1 bis 3, worin der Gehalt an phosphorhaltende Triglyzerid von 0,005 g bis 0,100 g per Verabreichungseinheit erstreckt.

5. Verwendung von einer diätetische Zubereitung für therapeutische Zweck nach einer der Ansprüche 1 bis 4, worin die inerte Stoffe sind die für die Verabreichung durch verdauungs Weg geeignet sind.

6. Verwendung von einer diätetische Zubereitung für therapeutische Zweck nach einer der Ansprüche 1 bis 5, worin das diätetische Zubereitung für therapeutische Zweck ausserdem Tocopherole enthält.

7. Verwendung von einer diätetische Zubereitung für therapeutische Zweck nach einer der Ansprüche 1 bis 6, worin der Gehalt an Tocopherole in der Zusammensetzung von 0,7 g bis 1,4 g erstreckt.

8. Verwendung von einer diätetische Zubereitung für therapeutische Zweck nach einer der Ansprüche 1 bis 7, worin das Zusammensetzung ausserdem Magnesium Salze enthält.

9. Verwendung von einer diätetische Zubereitung für therapeutische Zweck nach einer der Ansprüche 1 bis 7, worin der Gehalt an Magnesium Salz, der in Oxyd ausgedrückt ist, von 25 bis 35 g für 100 g erstreckt.

10. Verwendung von einer diätetische Zubereitung für therapeutische Zweck nach einer der vorhergehende Ansprüche worin die tägliche Verabreichung von 2 bis 6 Verabreichungseinheiten bei erwachsenen Menschen erstreckt.
